# EUROPEAN PATENT APPLICATION

(11) **EP 4 292 592 A1**
(43) Date of publication of application: **20.12.2023**
(21) Application number: 22753001.1
(22) Date of filing: 10.02.2022
(51) Int. Cl.: A61K 31/215, A61P 3/00, A61P 9/10, A61P 1/16, A23L 33/10

(54) **COMPOSITON FOR CONTROLLING SUGAR, CONTAINING ATYPICAL PKC ACTIVATOR**

(30) Priority: 15.02.2021 KR 20210019832
(71) Applicant: Industry-Academic Cooperation Foundation, Yonsei University, Seoul 03722 (KR); Arontier Co., Ltd., Seoul 06735 (KR)
(72) Inventor: KU, Cheol Ryong, Seoul 04422 (KR); KANG, Chan Woo, Seoul 04188 (KR); WANG, Eun Kyung, Seoul 04705 (KR); OH, Ju Hun, Seoul 03716 (KR); HONG, Zhenyu, Seoul 06300 (KR); SOHN, Insuk, Seoul 06341 (KR); KANG, Un Ho, Seoul 06373 (KR)
(74) Representative: Mewburn Ellis LLP
(86) International application number: PCT/KR2022/002035
(87) International publication number: WO 2022/173241

(57) **Abstract**

The present invention relates to a composition for regulating glucose that regulates the amount of glucose metabolized in the small intestine. According to the present invention, it is possible to improve glucose metabolism in the small intestine and to promote blood glucose excretion into feces, thus lowering blood glucose concentration, thereby preventing, alleviating or treating metabolic disease.

## Description

### Technical Field

The present invention relates to a composition for regulating glucose that regulates the amount of glucose metabolized in the small intestine, and more particularly, to a composition for regulating glucose containing an atypical PKC activator as an active ingredient, and a pharmaceutical composition for preventing or treating metabolic disease containing an atypical PKC activator as an active ingredient.

### Background Art

Diabetes mellitus is a chronic metabolic disease resulting from various inducing factors and characterized by long-lasting high blood glucose levels (hyperglycemia). Cells require insulin to use blood glucose, and insulin is secreted from the islets of Langerhans in the pancreas and has the effect of lowering blood glucose levels after meals. Insulin is a hormone that is secreted from beta cells of the pancreas and regulates carbohydrate and fat metabolism. Insulin also stimulates target cells (liver, muscle, and fat cells, etc.) to absorb and store glucose from the bloodstream. In normal people, insulin acts to convert blood glucose in the body into energy and lowers blood glucose levels. In contrast, in diabetic patients, insulin secretion does not occur normally, or even if insulin is secreted normally, problems with the insulin receptor may cause insulin to lose its ability to control blood glucose, resulting in high blood glucose levels. Diabetes can be broadly classified into two types. In type 1 diabetes or insulin-dependent diabetes mellitus (IDDM), patients produce little or no insulin, a hormone that regulates glucose utilization. Pancreatic beta cells are destroyed by an autoimmune mechanism, resulting in insulin deficiency. Therefore, glucose in the blood is not absorbed into the cells, leading to diabetes. In contrast, in type 2 diabetes or non-insulin-dependent diabetes mellitus (NIDDM), insulin is still produced in the body. This is acquired diabetes, accounting for more than 80% of all diabetes. Even though a sufficient amount of insulin is secreted from pancreatic beta cells, it is not absorbed into cells, resulting in a hyperglycemic state. Patients with type 2 diabetes are resistant to the effects of insulin, which stimulates glucose and lipid metabolism in the major insulin-sensitive tissues, which are muscle, liver and adipose tissues. This lack of responsiveness to insulin results in insufficient insulin-mediated activation of glucose uptake, oxidation and storage in muscle and inadequate insulin-mediated inhibition of lipolysis in adipose tissue and glucose production and secretion in the liver.

Diabetes affects more than 150 million people worldwide, and the number of diabetic patients has recently increased rapidly. According to data provided by the WHO, it is expected that more than 360 million diabetic patients will occur by 2030. In addition, even in Korea, as lifestyle changes and meat-oriented eating habits and lack of exercise increase, the incidence of diabetes patients is rapidly increasing, and the age of onset of diabetes is also decreasing. After being diagnosed with diabetes, diet is restricted to control blood glucose levels, quality of life is lowered, and the risk of various complications is caused. Therefore, the development of technology to control blood glucose levels within a normal range has continued. For example, Korean Patent No. 10-1983982 discloses technology for treating diabetes using desPro36exendin-4(1-39)-Lys6-NH2 and glitazone, and Korean Patent Application Publication No. 10-2006-0020548 discloses technology for treating diabetes using a crude polysaccharide extract of *Tremella fuciformis.* However, these related technologies are only intended to move a high concentration of glucose present in the blood to other parts in the body, and have a problem in that they cannot release glucose out of the body.

Accordingly, the present inventors have made extensive efforts to screen a drug that induces increased glucose absorption in the small intestine, and as a result, have screened prostratin and ingenol-3-angelate, which are PKC activators, as compounds having glucose regulation ability, and have found that glucose absorption in the small intestine of diabetic model mice to which the prostratin was administered increases and glucose excretion through the feces increases, thereby completing the present invention.

### Summary of the Invention

An object of the present invention is to provide a composition for regulating glucose that induces increased glucose absorption in the small intestine.

Another object of the present invention is to provide a composition for preventing or treating metabolic disease containing a glucose-regulating compound as an active ingredient.

Still another object of the present invention is to provide a functional food composition for preventing or alleviating metabolic disease containing a glucose-regulating compound as an active ingredient.

However, objects to be achieved by the present invention are not limited to the above-mentioned objects, and objects not mentioned herein will be clearly understood by those skilled in the art from the following description.

In order to achieve the above object, the present invention provides a composition for regulating glucose containing an atypical PKC activator as an active ingredient.

The present invention also provides a composition for preventing or treating metabolic disease containing an atypical PKC activator as an active ingredient.

The present invention also provides a method for preventing or treating metabolic disease comprising a step of administering an atypical PKC activator.

The present invention also provides the use of an atypical PKC activator for preventing or treating metabolic disease.

The present invention also provides the use of an atypical PKC activator in the manufacture of a medicament for preventing or treating metabolic disease.

The present invention also provides a functional food composition for preventing or alleviating metabolic disease containing an atypical PKC activator as an active ingredient.

### Brief Description of Drawings

FIG. 1 shows classification of PKC isoforms. PKCs are broadly classified into conventional or classical, novel, and atypical subfamilies, and FIG. 1 shows the gene names, protein names, domains, and ligands of each subfamily.
FIG. 2 shows the results of analyzing protein changes in cell lysates by Western blotting after treatment of IEC-6 and IEC-18 cells with each of prostratin and ingenol-3-angelate.
FIG. 3 shows the results of examining 2-DG uptake after treating IEC-6 and IEC-18 cells with each of prostratin and ingenol-3-angelate.
FIG. 4 shows the results of confirming the increase in activities of PKC zeta and GLUT1 after treating IEC-18 cells with each of prostratin and ingenol-3-angelate.
FIG. 5A shows the results of analyzing the expression of PKC zeta, p-GLUT1 and GLUT1 in PKC zeta-transfected IEC-6 cells and IEC-18 cells, FIG. 5B shows the result of analyzing the expression of PKC zeta, p-GLUT1 and GLUT1 in PKC zeta-transfected HEK293 cells, and FIG. 5C shows the result of analyzing 2-DG uptake in PKC zeta-transfected IEC-18 cells.
FIG. 6 shows the results of measuring glucose uptake after treating prostratin- or ingenol-treated EC-6 cells and IEC-18 cells with PKC zeta inhibitor (ZIP).
FIG. 7 shows the results of analyzing changes in the expression of p-GLUT1 and GLUT1 by Western blotting after transfecting the IEC-6 cell line with siRNA for each of PKCα, PKCδ, PKCζ and PKC 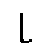 , and then treating each cell line with prostratin.
FIG. 8 shows the results of examining changes in 2-DG uptake after transfecting the IEC-6 cell line with siRNA for each of PKCα, PKCδ, PKCζ, and PKC 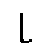 , and then treating each cell line with each of prostratin and ingenol.
FIG. 9A shows the results of performing an IP glucose tolerance test after single subcutaneous injection of ingenol or prostratin into C57BL6 mice and intraperitoneal administration of 2 g/kg of glucose to the mice, and FIG. 9B shows the results of performing an IP glucose tolerance test after administering prostratin to C57BL6 mice for 2 weeks and intraperitoneally administering 2 g/kg of glucose to the mice.
FIG. 10 shows the results of performing body weight measurement (left) and an intraperitoneal glucose tolerance test (right) after intraperitoneally injecting each of prostratin (0.5 mg/kg) and ingenol (1µg/kg) into diabetic model mice daily for 40 days.
FIG. 11A shows the results of radiography for 18FDG in the intestines harvested from diabetic model mice to which prostratin was administered for 1 month, FIG. 11B shows the result of measuring the amount of 18FDG in the fecal sample of the harvested intestine, and FIG. 11C shows the result of measuring the amounts of 18FDG absorption in the duodenum, jejunum, and ileum of the small intestine by a gamma counter.
FIG. 12 shows the results of immunohistochemically staining the duodenum, jejunum, and ileum with an antibody against PKC zeta after harvesting the small intestine from diabetic model mice to which prostratin was administered for 1 month. The left side shows the results of imaging with an optical microscope at 100x magnification, and the right side shows the results of imaging at 400x magnification.
FIG. 13 shows electron microscope images of the apical membrane portion of the lumen in the jejunum of the small intestine harvested from diabetic model mice to which prostratin was administered for 1 month.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, AI-based drug screening was conducted to select drugs that exhibit similar or superior effects to Hb-EGF, an EGFR ligand that induces increased glucose absorption in the small intestine, and as a result, selected prostratin and ingenol-3-angelate (hereinafter referred to as "ingenol") as candidate drugs. Both prostratin and ingenol-3-angelate act as activators of protein kinase C (PKC).

In one embodiment of the present invention, it was confirmed that, in intestinal epithelial cell lines (IEC-6 and IEC-18) treated with prostratin and ingenol, the expression of p-GLUT1 and GLUT1, proteins involved in glucose uptake in the cell membrane, increased and the uptake of 2-DG, a glucose analogue, also increased.

In another embodiment of the present invention, it was confirmed that, when prostratin was administered to diabetic model mice (db/db mice), intraperitoneal glucose tolerance increased, leading to improvement in glucose metabolism, and the body weight of the prostratin-administered mouse group decreased significantly.

Therefore, in one aspect, the present invention is directed to a composition for regulating glucose containing an atypical PKC activator as an active ingredient.

In the present invention, the atypical PKC may be PKC zeta (PKC ζ) or PKC iota (PKC 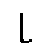 ).

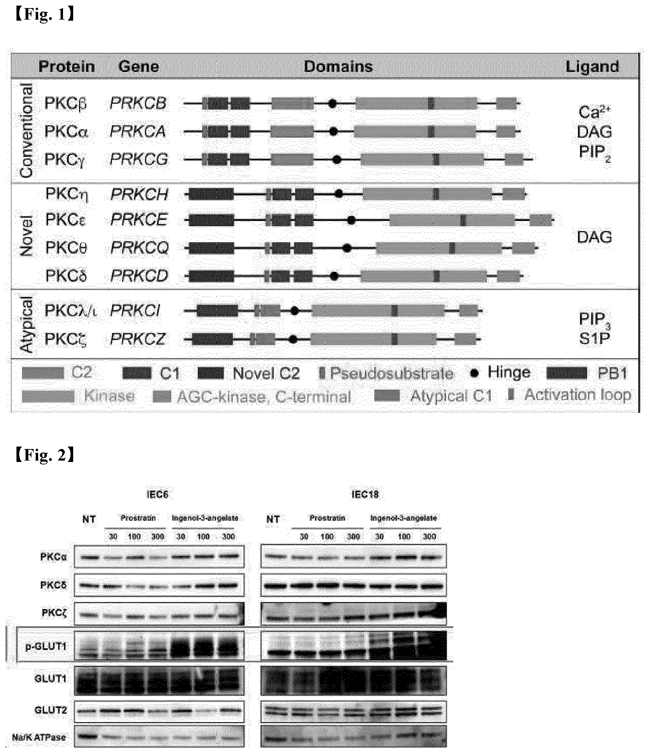

The PKC family is classified into three subfamilies: conventional or classic (PKCα, PKCβ, and PKCγ), novel (PKCδ, PKCε, PKCη, and PKCθ), and atypical (PKCζ and PKCλ/ 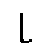 ) isoforms. The ligands that activate each isoform PKC are different depending on the subfamily. The classic isoform is activated by Ca2+, DAG, and PIP2, and the novel isoform is activated by DAG and lipid. The atypical PKCs have no C2 and C1 domains, and thus have the characteristic of not being activated by Ca2+ and DAG. In addition, it is known that the atypical PKCs interact with proteins such as polarity regulator (PAR6) because they contain a Phox and Bern 1 (PB1) domain (FIG. 1).

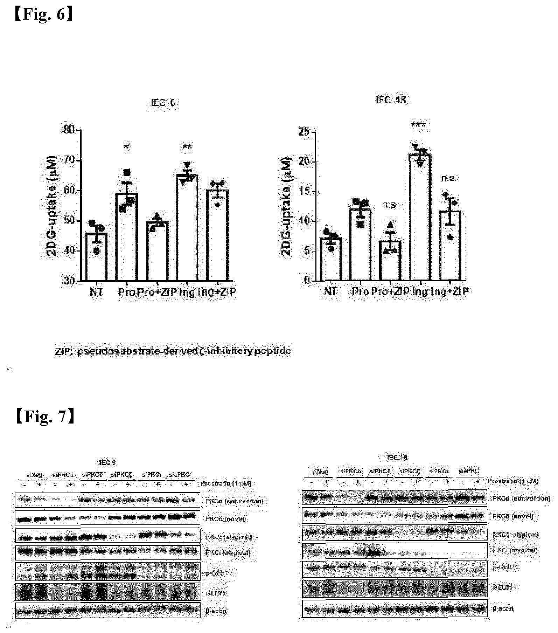

In one embodiment of the present invention, the IEC-6 cell line was transfected with siRNA for each of conventional PKC (PKCα), novel PKC (PKCδ) and atypical PKCs (PKCζ and PKC 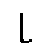 ), and after 48 hours, treated with prostratin (1 µM), and total protein was extracted and subjected to Western blotting. As a result, it was confirmed that the expression of GLUT1 (p-GLUT1), which was increased by prostratin in the negative control group (siNeg), did not increase in the cell group treated with siRNA for each of PKCζ and PKC 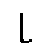 , which are atypical PKCs, even when the cell group was treated with prostratin (FIG. 7). Therefore, it was confirmed that PKCζ and PKC 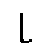 would play important roles in the mechanism of increasing the activity of GLUT1 (p-GLUT1) by prostratin.

In the present invention, regulating glucose may comprise regulating glucose absorption in the small intestine or glucose excretion.

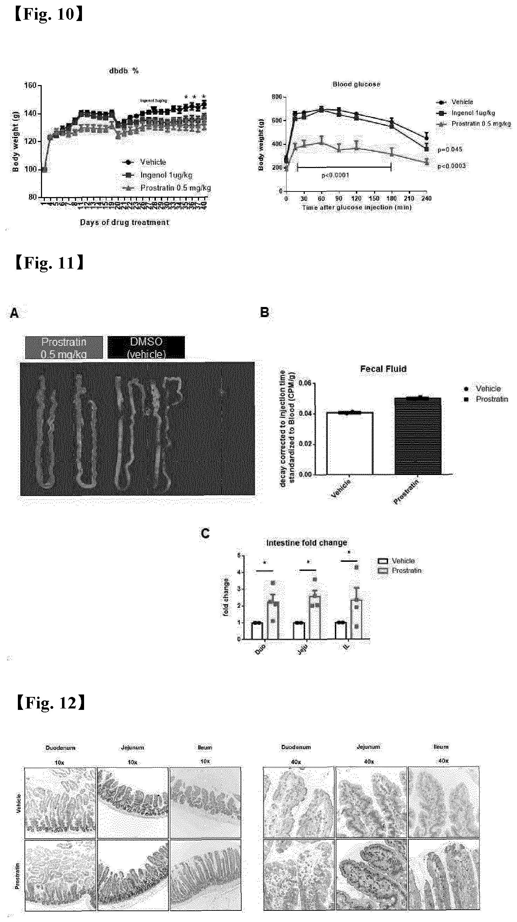

In one embodiment of the present invention, from the results of radiography of the intestines harvested from diabetic model mice (db/db mice) to which prostratin (0.5 mg/kg) was administered for 1 month, it was confirmed that glucose absorption in the small intestine from the db/db mice treated with prostratin was significantly higher than that in the vehicle group. In addition, it was confirmed that glucose excretion was higher in the fecal sample from the mice to which prostratin was administered (FIG. 11).

In the present invention, the atypical PKC activator may be selected from the group consisting of prostratin or a salt thereof, a prostratin derivative or a salt thereof, ingenol-3-angelate or a salt thereof, and an ingenol-3-angelate derivative or a salt thereof.

The "prostratin" is a compound having a structure of the following Formula 1, which is a modulator of protein kinase C, and is known to exhibit promising therapeutic potential for cancer and other diseases such as Alzheimer's disease, and it was reported that orally administered prostratin inhibited human pancreatic tumors (Wang, Man-Tzu et al., Cell. 16: 1237, 2015).

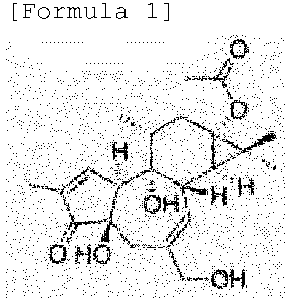

The "ingenol-3-angelate" that is used in the present invention has a structure of the following Formula 2, is also known as "ingenol mebutate", and is commercially available under the brand name "Picato". This is a substance found in the sap of the *Euphorbia peplus* plant and is known to induce cell death. A gel formulation of the drug has been approved by the US Food and Drug Administration (FDA) and the European Medicines Agency (EMA) for the topical treatment of actinic keratosis.

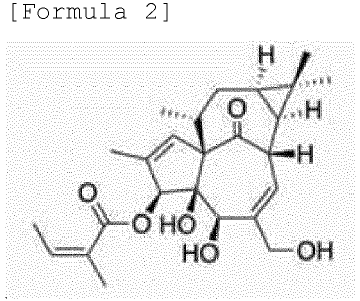

In the present invention, the composition for regulating glucose is capable of inducing blood glucose uptake into cells in the small intestine, preferably the duodenum, jejunum, and ileum of the small intestine, and blood glucose excretion through feces.

In the present invention, the "ileum" is the distal part of the small intestine and is located between the jejunum and the large intestine. The small intestine is a digestive organ located between the stomach and the large intestine and is anatomically composed of three parts: the duodenum, the jejunum, and the ileum. Normally, the small intestine of a human over the age of 5 is about 7 m long, which is 4 to 5 times longer than the large intestine, but the thickness thereof is 2.5 to 3 cm, which is much thinner than that of the large intestine (about 7.6 cm). The duodenum is about 25 to 30 cm long, and the jejunum which is the middle part of the small intestine is about 2.5 m long. The ileum is about 3.6 m long and is connected to the colon at the ileocecal valve. The ileocecal valve passes digested material into the large intestine and prevents reflux into the small intestine. As a result of digestion, nutrients are diffused and delivered into the blood through the villi in the small intestine. The mucous membrane in the small intestine has numerous wrinkles, and there are villi on the surface thereof. Usually, most of the chyme from the stomach is digested and absorbed in the duodenum and jejunum, which correspond to the beginning and middle of the small intestine. In contrast, it is known that digestion and absorption in the ileum are relatively low (Diabetes Management Interactive Case Study, 2016). The composition of the present invention has an effect of increasing glucose absorption in the duodenum, jejunum and ileum.

In addition, the composition for regulating glucose according to the present invention has the effect of not only promoting blood glucose excretion through feces but also improving glucose metabolism, and thus may be used to effectively prevent, alleviate, or treat metabolic disease.

Therefore, in another aspect, the present invention is directed to a composition for preventing or treating metabolic disease containing an atypical PKC activator as an active ingredient.

In the present invention, the atypical PKC may be PKC zeta (PKC ζ) or PKC iota (PKC 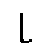 ).

In the present invention, regulating glucose may comprise regulating glucose absorption in the small intestine and glucose excretion.

In the present invention, the atypical PKC activator may be selected from the group consisting of prostratin or a salt thereof, a prostratin derivative or a salt thereof, ingenol-3-angelate or a salt thereof, and an ingenol-3-angelate derivative or a salt thereof.

In the present invention, the "metabolic disease" refers to a disease caused by excessive synthesis or accumulation of fat due to abnormal energy metabolism in the body resulting from various causes such as excessive energy intake or hormonal imbalance. Specifically, the metabolic disease may be obesity, diabetic disease, hypertension, hyperlipidemia, hypertriglyceridemia, hypercholesterolemia, fatty liver, or arteriosclerosis.

In the present invention, examples of the "diabetic disease" include, but are not limited to, diabetes, and complications caused by acute hyperglycemia, such as diabetic ketoacidosis, diabetic acidosis, diabetic xanthoma, diabetic amyotrophy, diabetic ketosis, diabetic coma, diabetic gastric disorder, diabetic gangrene, diabetic ulcer, diabetic complications, diabetic diarrhea, diabetic microangiopathy, diabetic uterine body sclerosis, diabetic cardiomyopathy, diabetic neuropathy, diabetic nephropathy, bullosis diabeticorum, diabetic cataract, diabetic dermopathy, diabetic scleredema, diabetic retinopathy, necrobiosis lipoidica diabeticorum, or diabetic blood circulation disorder.

In the present invention, "diabetes" is a disease caused by an increase in the concentration of glucose in the blood, and an increase in the duration of diabetes leads to a rapid increase in the risk of blindness due to chronic complications, end-stage renal failure, neurological diseases, lower extremity amputations, infectious diseases, and the like. In particular, as complications of diabetes, cerebrovascular disease and cardiovascular disease account for the largest share. It has been reported that about 3/4 of deaths from diabetes are due to diabetic complications, and the risk of death from cardiovascular complications also increases by 24% for every 10 years of diabetes duration. It has been reported that diabetic patients have a twice as high prevalence of coronary artery disease and an about three-fold higher prevalence of peripheral vascular disease compared to normal people. Various causes of atherosclerosis in diabetes are known, including hyperglycemia, abnormal lipid metabolism, hyperinsulinemia, hypertension, and changes in blood coagulation mechanisms. It is known that the pathogenesis of type 2 (non-insulin dependent) diabetes, which accounts for more than 95% of diabetes, is due to two causes, namely a complex disorder of insulin secretion disorder and insulin resistance. In other words, diabetes is a disease that shows chronic hyperglycemia symptoms due to this complex disorder.

In the present invention, the "diabetic ketoacidosis" is the most important acute metabolic complication that occurs in diabetic patients, and is a disease caused by loss of water and sugar and the excessive accumulation of acid metabolites in the bloodstream, which is caused by using fat rather than sugar for energy needed by the body. Diabetic ketoacidosis is caused by insulin resistance or insulin deficiency. When there is not enough insulin, glucose cannot enter the cells and accumulates in the blood. As a result, the cells do not receive glucose and thus use fat as an energy source. Fat metabolism produces fatty acids and glycerol, in which glycerol provides some energy to cells, but fatty acids are metabolized to ketones, resulting in acidosis. Acidosis increases the movement of potassium from the cells into the blood vessels, resulting in hyperkaluria due to diuretic action, which results in systemic potassium depletion.

In the present invention, "obesity" may mean a condition or disease with excessive body fat caused by energy imbalance, and refers to a disease in which excessive fat is synthesized or accumulated due to poor energy metabolism in the body for various reasons.

In still another aspect, the present invention is directed to a method for preventing or treating metabolic disease comprising a step of administering an atypical PKC activator.

In yet another aspect, the present invention is directed to the use of an atypical PKC activator for preventing or treating metabolic disease.

In still yet another aspect, the present invention is directed to the use of an atypical PKC activator in the manufacture of a medicament for preventing or treating metabolic disease.

In a further aspect, the present invention is directed to a functional food composition for preventing or alleviating metabolic disease containing an atypical PKC activator as an active ingredient.

In the present invention, the atypical PKC may be PKC zeta (PKC ζ) or PKC iota (PKC 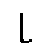 ).

In the present invention, regulating glucose may comprise regulating glucose absorption in the small intestine and glucose excretion.

In the present invention, the atypical PKC activator may be selected from the group consisting of prostratin or a salt thereof, a prostratin derivative or a salt thereof, ingenol-3-angelate or a salt thereof, and an ingenol-3-angelate derivative or a salt thereof.

In one embodiment of the present invention, "prevention" may include, without limitation, any action capable of blocking, suppressing or delaying symptoms caused by metabolic disease by using the composition of the present invention.

In one embodiment of the present invention, "alleviation" may include, without limitation, any action capable of alleviating or beneficially changing symptoms caused by metabolic diseases by using the composition of the present invention.

In one embodiment of the present invention, "treatment" refers to a series of actions performed to relieve and/or alleviate a target disease. With regard to the purposes of the present invention, treatment includes actions that improve glucose metabolism by significantly increasing the amount of glucose absorbed in the intestine.

In one embodiment of the present invention, "pharmaceutical composition" refers to a composition that is administered for a specific purpose. For the purposes of the present invention, the pharmaceutical composition of the present invention serves to prevent or treat diabetes or diabetic complications, and may contain a compound involved therein and a pharmaceutically acceptable carrier, excipient or diluent.

In addition, the pharmaceutical composition according to the present invention contains the active ingredient of the present invention in an amount of 0.1 to 50 wt% based on the total weight of the composition. Examples of carriers, excipients and diluents that may be contained in the composition of the present invention include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In one embodiment of the present invention, "administration" means introducing the composition of the present invention to a patient by any suitable method, and the composition of the present invention may be administered via any general route as long as it can reach the target tissue.

The composition may be administered orally, intraperitoneally, intravenously, intramuscularly, subcutaneously, intradermally, intranasally, intrapulmonarily, enterically, intracavitarily, intraperitoneally, or intrathecally, without being limited thereto. In the present invention, the effective amount may be determined depending on various factors, including the type of disease, the severity of the disease, the types and contents of the active ingredient and other ingredients contained in the composition, the type of formulation, the patient's age, body weight, general health condition, sex and diet, administration time, the route of administration, the secretion rate of the composition, the treatment period, and concurrently used drugs. In addition, the pharmaceutical composition of the present invention may be administered alone or together with other therapies known in the art, such as chemotherapy and surgery, for the treatment of a target metabolic disease. In addition, the pharmaceutical composition of the present invention may be administered in combination with other treatments designed to promote blood glucose lowering, for example, those well known in the art. Other standard delivery methods may also be used, such as biolistic delivery or ex *vivo* treatment.

In one embodiment of the present invention, the "food composition" is variously used for the prevention or alleviation of a target metabolic disease in the present invention. The food composition comprising the composition of the present invention as an active ingredient may be prepared in the form of various foods, such as beverages, gum, tea, vitamin complexes, powders, granules, tablets, capsules, confectionery, rice cakes, bread, etc. The food composition of the present invention may be safely used even when taken for a long period of time for preventive purposes because it is an improved food composed of existing food components with little toxicity and side effects. When the composition of the present invention is contained in a food composition, it may be added in an amount of 0.1 to 100 wt% based on the total weight. Here, when the food composition is prepared in the form of a beverage, there is no particular limitation except that the food composition is contained at the indicated ratio. In this case, the food composition may contain various flavoring agents or natural carbohydrates as additional ingredients as in conventional beverages. Examples of the natural carbohydrates include conventional sugars, such as monosaccharides (e.g., glucose, etc.), disaccharides (e.g., sucrose, etc.), polysaccharides (e.g., dextrin, cyclodextrin, etc.), and sugar alcohols such as xylitol, sorbitol, erythritol or the like. Examples of the flavoring agents include natural flavoring agents (thaumatin, stevia extract (e.g., rebaudioside A, glycyrrhizin, etc.) and synthetic flavoring agents (saccharin, aspartame In addition, the food of the present invention may contain various nutrients, vitamins, minerals (electrolytes), flavoring agents such as synthetic flavoring agents and natural flavoring agents, colorants, extenders, pectic acid and salts thereof, alginic acid and salts thereof, organic acids, protective colloidal thickeners, pH adjusting agents, stabilizers, preservatives, glycerin, alcohol, carbonizing agents that are used in carbonated beverages, etc. These components may be used independently or in combination. The proportion of these additives is generally selected from the range of 0.1 to 100 parts by weight based on 100 parts by weight of the composition of the present invention, without being limited thereto.

### [Examples]

Hereinafter, the present invention will be described in more detail with reference to examples. These examples are only for illustrating the present invention, and it will be apparent to those skilled in the art that the scope of the present invention is not to be construed as being limited by these examples.

### Example 1: Screening of drugs that increase glucose absorption in the ileum of the small intestine

In order to screen for drugs that have similar or superior effects to Hb-EGF, an EGFR ligand that induces increased glucose absorption in the ileum, Arontier's AI-based drug screening (Arontier, Korea) was performed.

Candidate compounds similar to Hb-EGF were screened using the REMEDY platform developed by Arontier Co., Ltd. (Lamb et al., Science, 313:1929-1935, 2006; Subramanian et al., Cell, 171:1437-1452, 2017).

As a result, prostratin and ingenol-3-angelate, which act as atypical PKC activators, were screened.

### Example 2: Evaluation of expression levels of GLUT transporters in small intestine cell lines after treatment with each of prostratin and ingenol-3-angelate

For culture of IEC-6 cells (ATCC CRL-1592) and IEC-18 cells (ATCC CRL-1589), which are intestinal epithelial cells, the cells were cultured using Dulbecco's modified Eagle medium (DMEM) supplemented with 10% fetal bovine serum (FBS) and penicillin-streptomycin at 37°C in a humidified atmosphere containing 10% CO₂ and 90% air, and were subcultured every 3 to 4 days. For the experiment, IEC-6 cells and IEC-18 cells were seeded in 6-well dishes, respectively, at a density of 2 × 10⁵ cells/dish. When the cell density reached 1.5 × 10⁵ cells/cm² after 6 days, the cultures were collected and used. Prostratin was purchased from Sigma-Aldrich (Cat. No. P0077) and used. Ingenol was purchased from Sigma-Aldrich (Cat. No. SML1318) and used.

IEC-6 cells and IEC-18 cells were treated with each of prostratin and ingenol at 30 nM, 100 nM, and 300 nM for 30 minutes, cell lysates were collected, and protein expression levels thereof were analyzed by Western blotting.

Western blot analysis was performed as follows.

Total cell protein lysates were prepared and subjected to Western blot analysis according to standard procedures. Briefly, cells were chilled on ice, washed twice with ice-cold phosphate-buffered saline, and lysed in buffer containing 1 mM phenylmethylsulfonyl fluoride and 1x protease inhibitor (Sigma-Aldrich). Protein concentration was measured with a Bradford assay kit (Bio-Rad, Hercules, CA, USA). Equal amounts of protein from the cell lysates were resolved by sodium dodecyl sulfate polyacrylamide gel electrophoresis and transferred to membranes which were then incubated with anti-PKCα (Cell Signaling Technology, USA), anti-PKCδ (Cell Signaling Technology, USA), anti-PKCζ (Cell Signaling Technology, USA), anti-p-GLUT1 (Abcam, GB), anti-GLUT1 (Abcam, GB) and anti-GLUT2 (Novus Biological, USA) as primary antibodies overnight at 4°C.

The blots were washed 3 times with TBST (Tris-buffered saline containing 0.05% Tween 20) and then incubated with horseradish peroxidase (HRP)-conjugated secondary antibodies for 1 hour at 25°C. As the secondary antibodies, donkey anti-rabbit IgG-HRP antibody (1:5000, Santa Cruz) and donkey anti-mouse IgG-HRP antibody (1:5000, Santa Cruz) were used. Immunoreactivity was detected with SuperSignal West Pico Chemiluminescent Substrate (Thermo Fisher Scientific, MA, USA).

As a result, as shown in FIG. 2, it was confirmed that GLUT1 phosphorylation in the IEC-6 and IEC-18 cells was promoted by each of ingenol-3-angelate and prostratin at all concentrations (30, 100, and 300 nM). It was confirmed that the expression of p-GLUT1 and GLUT1, which are glucose transporters, was increased by each of prostratin and ingenol-3-angelate when compared to Na/K ATPase, a protein quantitative marker.

### Example 3: Evaluation of intracellular glucose uptake in small intestine cell lines after treatment with each of prostratin and ingenol-3-angelate

IEC-6 cells and IEC-18 cells were treated with each of prostratin and ingenol (100 nM) for 10 minutes, and then uptake of 2-deoxy-D-glucose (2-DG) in the collected cell lysates was analyzed.

By measuring the glucose concentration, changes in glucose uptake (2-DG uptake) by treatment with each of prostratin and ingenol were determined.

The amount of glucose was measured after treatment with each of prostratin and ingenol in a low-concentration glucose medium and serum-free DMEM for 24 hours. Glucose levels in the IEC-6 and IEC-18 cells were detected using a Glucose Assay Kit (Cayman Chemical) according to the manufacturer's instructions. Absorbance signals were measured with a Gen5 spectrophotometer (BioTek, Winooski, VT, USA).

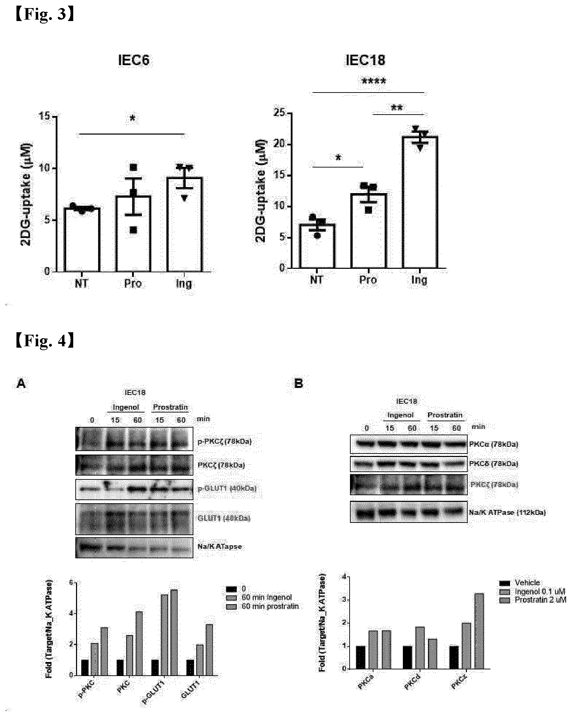

As a result, as shown in FIG. 3, it was confirmed that intracellular glucose (2-DG) uptake was increased by treatment with each of prostratin and ingenol.

### Example 4: Confirmation of activation of PKC and GLUT1 in small intestine cell line by each of prostratin and ingenol-3-angelate

After treatment of IEC-18 cells with each of prostratin (2 µM) and ingenol (0.1 µM) for 0, 15, and 60 minutes, the expression of PKC and GLUT1 proteins was analyzed by Western blotting.

As a result, as shown in FIG. 4, it was confirmed that each of prostratin and ingenol increased the activities of PKC zeta and GLUT1. In addition, it was confirmed that prostratin more rapidly translocated PKC and GLUT1 to the cell membrane than ingenol.

### Example 5: Confirmation that PKC zeta induces GLUT1-mediated glucose uptake in small intestine cell lines

Transfected cell lines were generated by transfection such that PKC zeta was transiently overexpressed in IEC-6 cells, IEC-18 cells, and HEK293 (ATCC CRL-1573) cells.

A rat PKC zeta-expressing plasmid (Korea Gene Bank) was subcloned into pCDNA3.1 (Addgene, USA). IEC-6 cells and IEC-18 cells were seeded in 6-well dishes, respectively, at a density of 0.5 × 10⁶ per dish one day before transfection. Cells were transfected with the expression plasmid using Polyjet transfection reagent (SignaGen) according to the manufacturer's instructions. Transfected cells were cultured for 48 hours at 37°C before lysis and treated with ingenol (0.1 µM). Cell lysates were collected for the empty vector-infected cell group as a control group, the PKC zeta-transfected cell group, the wild-type cell group treated with ingenol, and the transfected cell group treated with ingenol, and subjected to Western blotting.

In Western blotting, Na/K ATPase was used as a membrane loading control.

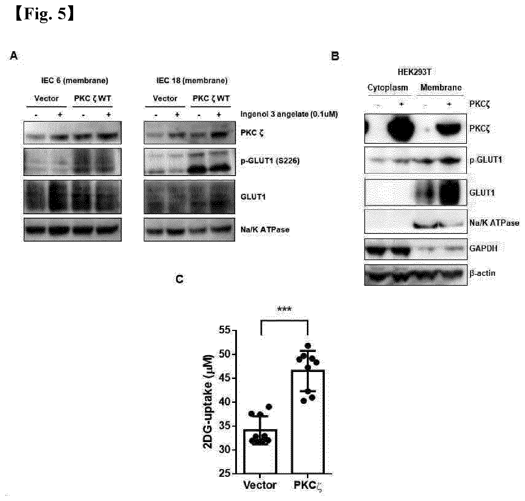

As a result, as shown in FIGS. 5A and 5B, it could be confirmed that PKC zeta was activated and translocated to the cell membrane and GLUT1 was translocated, in the ingenol-treated group compared to the empty vector-treated negative control group. In the PKC zeta-overexpressing cell line, p-GLUT1 and GLUT1 were more expressed even in the group not treated with ingenol than in the negative control group.

In addition, 2-DG uptake was measured in the transfected IEC-18 cell line overexpressing PKC zeta. As a result, as shown in FIG. 5C, it was confirmed that 2-DG uptake significantly increased in the PKC zeta-overexpressing IEC-18 cell line compared to the control group.

Therefore, it was confirmed that 2-DG uptake significantly increased in the cell line with high expression of atypical PKC.

### Example 6: Confirmation of inhibition of glucose uptake effect of each of prostratin and ingenol by treatment with PKC zeta inhibitor

EC-6 cells and IEC-18 cells, in which atypical PKC was activated by treatment with each of prostratin (100 nM) and ingenol (100 nM) were treated with 1 µM of ZIP (pseudosubstrate-derived PKCζ-inhibitory peptide, R&D system, USA) that specifically inhibits PKC zeta, and then glucose uptake therein was measured.

As a result, as shown in FIG. 6, it was confirmed that, when IEC-6 or IEC-18 cells were treated with each of prostratin (100 nM) and ingenol (100 nM), 6-phosphate glucose in the cells increased, but when the cells were treated with a combination of prostratin or ingenol and the PKC zeta inhibitor ZIP, the uptake of 6-phosphate glucose in the cells decreased to that in the negative control group (NT).

### Example 7: Identification of the role of atypical PKC (zeta +iota) in prostratin-mediated GLUT1 activation

To identify which PKC subtypes induce GLUT1 activation by each of prostratin and ingenol, the IEC-6 cell line was transfected with 50 nM of siRNA for each of conventional PKC (PKCα), novel PKC (PKCδ), and atypical PKCs (PKCζ and PKC 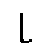 ) using RNAiMax (Invitrogen). After 48 hours, the cells were treated with prostratin (1 µM), and total protein was extracted and subjected to Western blotting.

The siRNA sequences used are as follows.
PKCα siRNA: GGGAUGUCAGAGAGCAUGCCUUCUU (SEQ ID NO: 1)
PKCδ siRNA: CUCACAGUACUUCCUCUGU (SEQ ID NO: 2)
PKCζ siRNA: GCUGAGAUCUGUAUCGCUCUCAACU (SEQ ID NO: 3)
PKC 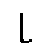 siRNA: GGACAAUGUACUGCUAGACUCUGAA (SEQ ID NO: 4)

As a result, as shown in FIG. 7, it was confirmed that the expression of GLUT1 (p-GLUT1), which was increased in the negative control group (siNeg) by prostratin, was increased in the cell groups treated with siRNAs for atypical PKCs (PKCζ and PKC 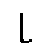 ) even when the cell groups were treated with prostratin.

Therefore, it was confirmed that PKCζ and PKC 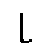 play important roles in the mechanism of increasing the activity of GLUT1 (p-GLUT1) by prostratin.

### Example 8: Identification of the role of atypical PKC (zeta + iota) in the increase in glucose uptake by each of prostratin and ingenol

In order to identify which PKC subtypes induce increased glucose uptake (2DG uptake) in IEC cells by treatment with each of prostratin and ingenol, the IEC-6 cell line was transfected with siRNA for each of conventional PKC (PKCα), novel PKC (PKCδ), and atypical PKCs (PKCζ and PKC 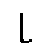 ) as described in Example 7. After 48 hours, the cells were treated with prostratin (1 µM) or ingenol (100 nM), and 2DG uptake therein was analyzed.

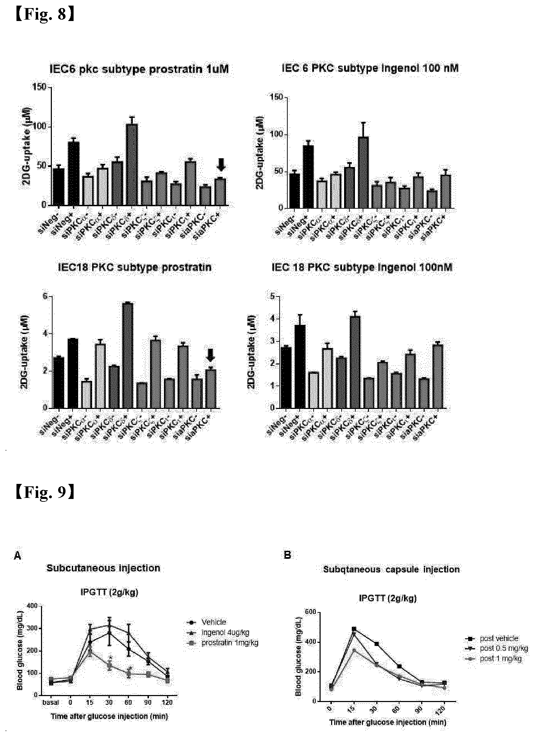

As a result, as shown in FIG. 8, it was confirmed that 2DG uptake in the negative control group (siNeg) was increased by treatment with each of prostratin and ingenol, but 2DG uptake in the atypical PKC (PKCζ and PKC 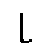 )-knockdown group was not increased even by treatment with each of prostratin and ingenol.

Therefore, it was confirmed that both PKCζ and PKC 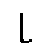 play an important role in glucose uptake induced by treatment with each of prostratin and ingenol.

### Example 9: Evaluation of glucose metabolism improvement effect of administration of each of prostratin and ingenol in mouse animal model

### 9-1: Confirmation of glucose metabolism improvement by single administration

After 12 C57BL6 mice were fasted for 12 hours, fasting blood glucose was measured by performing an intraperitoneal glucose test. Thereafter, ingenol (4 ug/kg) and prostratin (1 mg/kg) was injected subcutaneously once into the ingenol-administered group (4 mice) and the prostratin-administered group, respectively, and 2 g of glucose per kg of mouse (2 g/kg glucose) was diluted in physiological saline and intraperitoneally administered to each mouse. Thereafter, an IP glucose tolerance test was performed at 15 minutes, 30 minutes, 60 minutes, 90 minutes, and 120 minutes.

As a result, as shown in FIG. 9A, it was confirmed that glucose metabolism was significantly improved in the prostratin-administered group compared to the negative control group (Vehicle) at 30 and 60 minutes, and glucose metabolism was also improved in the ingenol-administered group compared to the negative control group.

### 9-2: Confirmation of improvement in glucose metabolism after 2 weeks of administration

Prostratin was administered to C57BL6 mice at concentrations of 0 mg/kg, 0.5 mg/kg, and 1 mg/kg for 2 weeks using an osmotic pump, followed by fasting for 12 hours. Then, 2 g of glucose per kg of mouse (2 g/kg glucose) was diluted in physiological saline and intraperitoneally administered to each mouse. Thereafter, an IP glucose tolerance test was performed at 15 minutes, 30 minutes, 60 minutes, 90 minutes, and 120 minutes.

As a result, as shown in FIG. 9B, it was confirmed that all of the area values under the glucose change curve for 2 hours were improved.

### Example 10: Evaluation of the effect of administration of each of prostratin and ingenol on improvement in body weight and glucose metabolism in diabetic model mice

Each of prostratin (0.5 mg/kg) and ingenol (1 ug/kg) was intraperitoneally injected daily for 40 days into db/db mice (Jackson Laboratory), known as a representative diabetes model due to deficiency of the leptin receptor, and then the body weight of each mouse was measured.

As a result, as shown in FIG. 10, it was confirmed that, after the concentration of ingenol was increased to 2 µg/kg on day 28 of administration, weight loss on day 30 showed statistical significance. In the group to which 0.5 mg/kg of prostratin was administered, weight loss on and after day 11 of administration showed statistical significance compared to that in the control group (vehicle group).

On day 17 after drug administration, an intraperitoneal glucose tolerance test (IPGTT) was performed. FIG. 10 shows the results of performing 2 g/kg IPGTT 15 minutes after injection of each of prostratin and ingenol after fasting for 12 hours before IPGTT. In the group to which 1 ug/kg of ingenol was administered, the glucose improvement effect appeared at 240 minutes after glucose administration. It was confirmed that, in the group to which prostratin (0.5 mg/kg) was administered, glucose improvement was superior to that in the control group (vehicle group) over the entire period of 30 to 240 minutes.

### Example 11: Evaluation of the effect of administration of prostratin on improvement in glucose absorption and excretion in diabetic model mice

The intestines of db/db mice (Jackson Laboratory) to which 0.5 mg/kg of prostratin had been administered for 1 month were harvested and radiographed for 18FDG (fluorodeoxyglucose, DuChemBio). 18FDG was purchased from DuChemBio and 200 µCi was injected into each mouse via the tail vein.

In addition, 18FDG in a fecal sample obtained by washing the harvested small intestine with 5 mL of PBS was measured, and 18FDG uptake of the duodenum, jejunum, and ileum of the small intestine was measured with a gamma counter.

As a result, as shown in FIG. 11A, it was confirmed from the radiographs that glucose absorption in the small intestine of the db/db mouse treated with prostratin was significantly higher than that in the control group (vehicle group).

In addition, as shown in FIG. 11B, as a result of examining glucose excretion in the fecal samples, it was confirmed that glucose excretion was higher in the fecal fluid from the mouse to which prostratin was administered. In addition, as shown in FIG. 11C, glucose absorption in all of the duodenum, jejunum, and ileum of the small intestine was more than doubled.

### Example 12: Evaluation of the effect of administration of prostratin on translocation of atypical PKC to membrane in jejunum and ileum of diabetic model mice

After administration of prostratin (0.5 mg/kg) or vehicle to db/db mice for 1 month, the small intestine was harvested, and the duodenum, jejunum, and ileum were fixed with formalin, subjected to immunohistochemical staining with anti-PKC zeta antibody (Abcam, USA), and imaged with an optical microscope at 100x and 400x magnifications.

As a result, as shown in FIG. 12, it could be confirmed that, in the duodenum, there was no significant difference between the prostratin-administered group and the control group (vehicle), and in the jejunum and ileum, the location of PKC zeta in the prostratin-administered group tended to be concentrated in the apical membrane of the lumen from the cytoplasm.

### Example 13: Electron microscopic observation of the lumen of the jejunum of prostratin-administered diabetic model mice

After administration of prostratin (0.5 mg/kg) or vehicle to db/db mice for 1 month, the small intestine was harvested, and the apical membrane portion of the lumen in the jejunum was observed with an electron microscope.

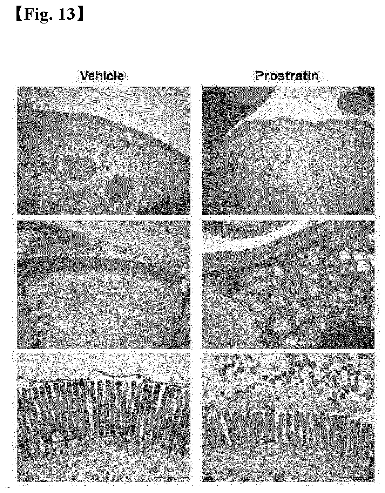

As a result, as shown in FIG. 13, it was confirmed that the glycocalyx near the microvilli of the jejunum of the prostratin-administered group was secreted more than that in the control group (vehicle), and the number of mitochondria in the small intestine cells of the prostratin-administered group was increased, indicating that intracellular metabolism actively occurred.

### Industrial Applicability

According to the present invention, it is possible to improve glucose metabolism in the small intestine and to promote blood glucose excretion into feces, thus lowering blood glucose concentration, thereby preventing, alleviating or treating metabolic disease.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence Listing Free Text

Electronic file attached

## Claims

1. A composition for regulating glucose comprising an atypical PKC activator as an active ingredient.

2. The composition according to claim 1, wherein the PKC is PKC zeta (PKC ζ) or PKC iota (PKC 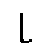 ).

3. The composition according to claim 1, wherein the regulating the glucose comprises regulating glucose absorption in the small intestine or glucose excretion.

4. The composition according to claim 1, wherein the atypical PKC activator is selected from the group consisting of prostratin or a salt thereof, a prostratin derivative or a salt thereof, ingenol-3-angelate or a salt thereof, and an ingenol-3-angelate derivative or a salt thereof.

5. A composition for preventing or treating metabolic diseases comprising an atypical PKC activator as an active ingredient.

6. The composition according to claim 5, wherein the PKC is PKC zeta (PKC ζ) or PKC iota (PKC 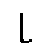 ).

7. The composition according to claim 5, which regulates glucose absorption in the small intestine.

8. The composition according to claim 5, wherein the atypical PKC activator is selected from the group consisting of prostratin or a salt thereof, a prostratin derivative or a salt thereof, ingenol-3-angelate or a salt thereof, and an ingenol-3-angelate derivative or a salt thereof.

9. The composition according to claim 5, which regulates glucose absorption in the small intestine or glucose excretion.

10. The composition according to claim 5, wherein the metabolic disease is diabetic disease, obesity, hypertension, hyperlipidemia, hypertriglyceridemia, hypercholesterolemia, arteriosclerosis, or fatty liver disease.

11. The composition according to claim 10, wherein the diabetic disease is selected from the group consisting of diabetes, diabetic ketoacidosis, diabetic acidosis, diabetic xanthoma, diabetic amyotrophy, diabetic ketosis, diabetic coma, diabetic gastric disorder, diabetic gangrene, diabetic ulcer, diabetic complications, diabetic diarrhea, diabetic microangiopathy, diabetic uterine body sclerosis, diabetic cardiomyopathy, diabetic neuropathy, diabetic nephropathy, bullosis diabeticorum, diabetic cataract, diabetic dermopathy, diabetic scleredema, diabetic retinopathy, necrobiosis lipoidica diabeticorum, and diabetic blood circulation disorder.

12. A functional food composition for preventing or alleviating metabolic disease comprising an atypical PKC activator as an active ingredient.

13. The functional food composition according to claim 12, which regulates sugar absorption in the small intestine or glucose excretion.

14. The functional food composition according to claim 13, wherein the atypical PKC activator is selected from the group consisting of prostratin or a salt thereof, a prostratin derivative or a salt thereof, ingenol-3-angelate or a salt thereof, and an ingenol-3-angelate derivative or a salt thereof.
